# EUROPEAN PATENT APPLICATION

(11) **EP 2 329 842 A2**
(43) Date of publication of application: **08.06.2011**
(21) Application number: 10182396.1
(22) Date of filing: 11.05.2001
(51) Int. Cl.: A61K 39/00, A61P 3/00, A61P 3/10

(54) **Interleukin-1 inhibitors in the treatment of diseases**

(30) Priority: 12.05.2000 US 203881 P; 01.08.2000 US 222422 P
(62) Divisional of application: 10006449.2
(71) Applicant: Immunex Corporation, Seattle, WA 98101 (US)
(72) Inventor: Sims, John E., Seattle, WA 98105 (US); O'Neal, Larry F., Pleasant SC 29464 (US); Connor, Timothy, Madison, WI 53719 (US); Hayes, Ann F., Seattle, WA 98144 (US)
(74) Representative: Lee, Nicholas John

(57) **Abstract**

The invention pertains to methods for treating medical disorders characterized by elevated levels or abnormal expression of IL-1 by administering an IL-1 antagonist, such as soluble type II IL-1 receptor.

SEQUENCE LISTING

<110> IMMUNEX CORPORATION
Sims, John
Connor, Timothy
Hayes, F. Ann
O'Neal, Larry

<120> INTERLEUKIN-1 INHIBITORS IN THE TREATMENT OF DISEASES

<130> P34970EP3

<140>
<141> 2001-05-11

<150> 60/203,881
<151> 2000-05-12

<150> 60/222,422
<151> 2000-08-01

<160> 2

<170> PatentIn version 3.0

<210> 1
<211> 1357
<212> DNA
<213> Homo sapiens

<220>
<221> CDS

<222>  (154)..(1347)

<220>

<221>  mat_peptide

<222>  (193)..()

<220>

<221>  sig_peptide

<222>  (154)..(192)

<400>  1
ctggaaaata cattctgcta ctcttaaaaa ctagtgacgc tcatacaaat caacagaaag      60

agcttctgaa ggaagacttt aaagctgctt ctgccacgtg ctgctgggtc tcagtcctcc     120

acttcccgtg tcctctggaa gttgtcagga gca atg ttg cgc ttg tac gtg ttg      174
                                     Met Leu Arg Leu Tyr Val Leu
                                                 -10

gta atg gga gtt tct gcc ttc acc ctt cag cct gcg gca cac aca ggg      222
Val Met Gly Val Ser Ala Phe Thr Leu Gln Pro Ala Ala His Thr Gly
    -5              -1  1               5                   10

gct gcc aga agc tgc cgg ttt cgt ggg agg cat tac aag cgg gag ttc      270
Ala Ala Arg Ser Cys Arg Phe Arg Gly Arg His Tyr Lys Arg Glu Phe
                15                  20                  25

agg ctg gaa ggg gag cct gta gcc ctg agg tgc ccc cag gtg ccc tac      318
Arg Leu Glu Gly Glu Pro Val Ala Leu Arg Cys Pro Gln Val Pro Tyr
            30                  35                  40

tgg ttg tgg gcc tct gtc agc ccc cgc atc aac ctg aca tgg cat aaa      366
Trp Leu Trp Ala Ser Val Ser Pro Arg Ile Asn Leu Thr Trp His Lys
        45                  50                  55

aat gac tct gct agg acg gtc cca gga gaa gaa gag aca cgg atg tgg      414
Asn Asp Ser Ala Arg Thr Val Pro Gly Glu Glu Glu Thr Arg Met Trp
    60                  65                  70

gcc cag gac ggt gct ctg tgg ctt ctg cca gcc ttg cag gag gac tct      462
Ala Gln Asp Gly Ala Leu Trp Leu Leu Pro Ala Leu Gln Glu Asp Ser
75                  80                  85                  90

ggc acc tac gtc tgc act act aga aat gct tct tac tgt gac aaa atg      510
Gly Thr Tyr Val Cys Thr Thr Arg Asn Ala Ser Tyr Cys Asp Lys Met
                95                  100                 105

tcc att gag ctc aga gtt ttt gag aat aca gat gct ttc ctg ccg ttc      558
Ser Ile Glu Leu Arg Val Phe Glu Asn Thr Asp Ala Phe Leu Pro Phe
            110                 115                 120

atc tca tac ccg caa att tta acc ttg tca acc tct ggg gta tta gta      606

Ile Ser Tyr Pro Gln Ile Leu Thr Leu Ser Thr Ser Gly Val Leu Val
        125                 130                 135

tgc cct gac ctg agt gaa ttc acc cgt gac aaa act gac gtg aag att      654
Cys Pro Asp Leu Ser Glu Phe Thr Arg Asp Lys Thr Asp Val Lys Ile
    140                 145                 150

caa tgg tac aag gat tct ctt ctt ttg gat aaa gac aat gag aaa ttt      702
Gln Trp Tyr Lys Asp Ser Leu Leu Leu Asp Lys Asp Asn Glu Lys Phe
155                 160                 165                 170

cta agt gtg agg ggg acc act cac tta ctc gta cac gat gtg gcc ctg      750
Leu Ser Val Arg Gly Thr Thr His Leu Leu Val His Asp Val Ala Leu
                175                 180                 185

gaa gat gct ggc tat tac cgc tgt gtc ctg aca ttt gcc cat gaa ggc      798
Glu Asp Ala Gly Tyr Tyr Arg Cys Val Leu Thr Phe Ala His Glu Gly
            190                 195                 200

cag caa tac aac atc act agg agt att gag cta cgc atc aag aaa aaa      846
Gln Gln Tyr Asn Ile Thr Arg Ser Ile Glu Leu Arg Ile Lys Lys Lys
        205                 210                 215

aaa gaa gag acc att cct gtg atc att tcc ccc ctc aag acc ata tca      894
Lys Glu Glu Thr Ile Pro Val Ile Ile Ser Pro Leu Lys Thr Ile Ser
    220                 225                 230

gct tct ctg ggg tca aga ctg aca atc ccg tgt aag gtg ttt ctg gga      942
Ala Ser Leu Gly Ser Arg Leu Thr Ile Pro Cys Lys Val Phe Leu Gly
235                 240                 245                 250

acc ggc aca ccc tta acc acc atg ctg tgg tgg acg gcc aat gac acc      990
Thr Gly Thr Pro Leu Thr Thr Met Leu Trp Trp Thr Ala Asn Asp Thr
                255                 260                 265

cac ata gag agc gcc tac ccg gga ggc cgc gtg acc gag ggg cca cgc     1038
His Ile Glu Ser Ala Tyr Pro Gly Gly Arg Val Thr Glu Gly Pro Arg
            270                 275                 280

cag gaa tat tca gaa aat aat gag aac tac att gaa gtg cca ttg att     1086
Gln Glu Tyr Ser Glu Asn Asn Glu Asn Tyr Ile Glu Val Pro Leu Ile
        285                 290                 295

ttt gat cct gtc aca aga gag gat ttg cac atg gat ttt aaa tgt gtt     1134
Phe Asp Pro Val Thr Arg Glu Asp Leu His Met Asp Phe Lys Cys Val
    300                 305                 310

gtc cat aat acc ctg agt ttt cag aca cta cgc acc aca gtc aag gaa     1182
Val His Asn Thr Leu Ser Phe Gln Thr Leu Arg Thr Thr Val Lys Glu
315                 320                 325                 330

gcc tcc tcc acg ttc tcc tgg ggc att gtg ctg gcc cca ctt tca ctg     1230
Ala Ser Ser Thr Phe Ser Trp Gly Ile Val Leu Ala Pro Leu Ser Leu
                335                 340                 345

gcc ttc ttg gtt ttg ggg gga ata tgg atg cac aga cgg tgc aaa cac     1278
Ala Phe Leu Val Leu Gly Gly Ile Trp Met His Arg Arg Cys Lys His
            350                 355                 360

aga act gga aaa gca gat ggt ctg act gtg cta tgg cct cat cat caa     1326
Arg Thr Gly Lys Ala Asp Gly Leu Thr Val Leu Trp Pro His His Gln
        365                 370                 375

gac ttt caa tcc tat ccc aag tgaaataaat                                    1357
Asp Phe Gln Ser Tyr Pro Lys
    380                 385

<210>  2

<211>  398

<212>  PRT

<213>  Homo sapiens

<400>  2

Met Leu Arg Leu Tyr Val Leu Val Met Gly Val Ser Ala Phe Thr Leu
            -10                 -5              -1  1

Gln Pro Ala Ala His Thr Gly Ala Ala Arg Ser Cys Arg Phe Arg Gly
    5                   10                  15

Arg His Tyr Lys Arg Glu Phe Arg Leu Glu Gly Glu Pro Val Ala Leu
20                  25                  30                  35

Arg Cys Pro Gln Val Pro Tyr Trp Leu Trp Ala Ser Val Ser Pro Arg
                40                  45                  50

Ile Asn Leu Thr Trp His Lys Asn Asp Ser Ala Arg Thr Val Pro Gly
            55                  60                  65

Glu Glu Glu Thr Arg Met Trp Ala Gln Asp Gly Ala Leu Trp Leu Leu
        70                  75                  80

Pro Ala Leu Gln Glu Asp Ser Gly Thr Tyr Val Cys Thr Thr Arg Asn
    85                  90                  95

Ala Ser Tyr Cys Asp Lys Met Ser Ile Glu Leu Arg Val Phe Glu Asn
100                 105                 110                 115

Thr Asp Ala Phe Leu Pro Phe Ile Ser Tyr Pro Gln Ile Leu Thr Leu
                120                 125                 130

Ser Thr Ser Gly Val Leu Val Cys Pro Asp Leu Ser Glu Phe Thr Arg
            135                 140                 145

Asp Lys Thr Asp Val Lys Ile Gln Trp Tyr Lys Asp Ser Leu Leu Leu
        150                 155                 160

Asp Lys Asp Asn Glu Lys Phe Leu Ser Val Arg Gly Thr Thr His Leu
    165                 170                 175

Leu Val His Asp Val Ala Leu Glu Asp Ala Gly Tyr Tyr Arg Cys Val
180                 185                 190                 195

Leu Thr Phe Ala His Glu Gly Gln Gln Tyr Asn Ile Thr Arg Ser Ile
                200                 205                 210

Glu Leu Arg Ile Lys Lys Lys Lys Glu Glu Thr Ile Pro Val Ile Ile
            215                 220                 225

Ser Pro Leu Lys Thr Ile Ser Ala Ser Leu Gly Ser Arg Leu Thr Ile
        230                 235                 240

Pro Cys Lys Val Phe Leu Gly Thr Gly Thr Pro Leu Thr Thr Met Leu
    245                 250                 255

Trp Trp Thr Ala Asn Asp Thr His Ile Glu Ser Ala Tyr Pro Gly Gly
260                 265                 270                 275

Arg Val Thr Glu Gly Pro Arg Gln Glu Tyr Ser Glu Asn Asn Glu Asn
                280                 285                 290

Tyr Ile Glu Val Pro Leu Ile Phe Asp Pro Val Thr Arg Glu Asp Leu
            295                 300                 305

His Met Asp Phe Lys Cys Val Val His Asn Thr Leu Ser Phe Gln Thr
        310                 315                 320

Leu Arg Thr Thr Val Lys Glu Ala Ser Ser Thr Phe Ser Trp Gly Ile
    325                 330                 335

Val Leu Ala Pro Leu Ser Leu Ala Phe Leu Val Leu Gly Gly Ile Trp
340                 345                 350                 355

Met His Arg Arg Cys Lys His Arg Thr Gly Lys Ala Asp Gly Leu Thr
                360                 365                 370

Val Leu Trp Pro His His Gln Asp Phe Gln Ser Tyr Pro Lys
            375                 380                 385

## Description

### BACKGROUND OF THE INVENTION

### Field of the Invention

The invention pertains to methods for treating certain diseases and disorders associated with inflammatory and immunoregulatory responses, More particularly, the present invention involves treating diseases characterized by IL-1 production by administering an Il-1 inhibitor, in particular type II IL-1R, to an individual afflicted with such a disease.

### Description of Related Art

The Interleukin-1 (IL-1) pathway is a cellular signaling pathway that plays a crucial role in the mammalian inflammatory response and is associated with a wide range of immunologic, metabolic, physiological and hematopoietic activities. The IL-1 family includes three structurally related cytokines: IL-1 alpha, IL-1 beta and IL-1 receptor antagonist (IL-1ra). Of the three, IL-1 alpha and IL-1 beta are proinflammatory agonists while IL-1 receptor antagonist (IL-1ra) functions to block IL-1 alpha and IL-1 beta activity. All known biological functions of IL-1 are mediated through the type 1IL-1R. 1L-1 alpha, 1L-1 beta and IL-1ra bind the type I IL-1R with high affinity. In contrast, IL-1 beta binds the type II IL-1R with high affinity and IL-1 alpha and IL-1ra bind the type II IL-1R with a low affinity. The type II IL-1R has a severely truncated cytoplasmic domain and upon binding to IL-1 does not transduce signal to a cell, but instead is involved in regulating an IL-1-mediated response by acting as a decoy receptor.

IL-1 production is triggered by infections, microbial toxins, inflammatory agents and allergic reactions. Overall the main functions of IL-1 is to regulate the amplitude and duration of the immune and inflammatory response at the sites of inflammation or allergic immune reaction. When excess IL-1. is produced or IL-1 expression is not appropriately regulated disease states can develop. Accordingly, IL-1 has been implicated in a variety of inflammatory and immunoregulatory diseases and conditions. It has been proposed that a systemic or localized excess of IL-1 contributes to the incidence of numerous medical disorders, Further to this proposal, it has been shown that IL-1ra, which blocks IL-1 alpha and IL-1 beta activity, has varying degrees of efficacy in treating some diseases thought to be mediated by IL-1 signaling. For example, a peptidomimetic that binds IL-1R and blocks IL-1 binding is reportedly clinically useful for suppressing IL-1 (Yanofsky, S.D. et al. Proc Natl Acad Scie USA 93(14):7381-6,1996; Akeson A.L. et al. J Biol Chem. 271(48):30517-23), 1996). Additionally, inhibitors of Interleukin-1 Converting Enzyme (ICE), an essential component in the formation of active IL-1 beta, are thought to be effective therapeutics for treating disease states associated with IL-1 activity. Further, a peptidomimetic that binds IL-1R and blocks IL-1 binding is reportedly clinically useful for suppressing IL-1 (Yanofsky, S.D. et al. Proc Natl Acad Scie USA 93(14):7381-6,1996; Akeson A.L. et al. J Biol Chem. 271(48):30517-23, 1996),

It has been suggested that the suppression of IL-1 might be beneficial in patients suffering from various disorders characterized by abnormal or excessive IL-1 expression or IL-1 activity. The IL-1ra and ICE inhibitors have met with limited degrees of success as therapeutics for diseases associated with IL-1 activity. Although progress has been made in devising effective treatment for such diseases, improved medicaments and methods of treatment are needed.

### SUMMARY OF THE INVENTI0N

Provided herein are methods for treating medical disorders associated with IL-1 mediated inflammatory reactions or IL-1 mediated immunoregulatory reactions. The methods of the present invention include administering an IL-1 antagonist, or IL-1 inhibitor, that inhibits IL-1 inflammatory or immunoregulatory signaling, to an individual afflicted with an inflammatory or immunoregulatory disease mediated by IL-1. More particularly, the present invention involves administering an IL-1 antagonist such as type II IL-1 receptor, to such an individual, for a period of time sufficient to induce a sustained improvement in the patient's condition.

### DETAILED DESCRIPTION OF THE INVENTION

The present invention provides methods for treating an individual including a human, who is suffering from a medical disorder that is associated with IL-1 mediated inflammatory reactions or IL-1 mediated immunoregulatory reactions. For purposes of this disclosure, the terms "illness," "disease," "medical condition" or "abnormal condition" are used interchangeably with the term "medical disorder."

The subject methods involve administering to the patient an IL-1 antagonist or IL-1 inhibitor that is capable of reducing the effective amount of endogenous biologically active IL-1, such as by reducing the amount of IL-1, or IL-1 beta produced, or by preventing the binding of IL-1 to its cell surface receptor type I IL-1R. Such antagonists include receptor-binding peptide fragments of IL-1, antibodies directed against IL-1 or IL-1 beta or IL-1 receptor type I, and recombinant proteins composing all or portions of receptors for IL-1 or modified variants thereof, including genetically-modified muteins, multimeric forms and sustained-release formulations. Particular antagonists include IL-1ra polypeptides, IL-1 beta converting enzyme (ICE) inhibitors, antagonistic type I IL-1 receptor antibodies, IL-1 binding forms of type I IL-1 receptor and type II IL-1 receptor, antibodies to IL-1, including IL-1 alpha and IL-1 beta and other IL-1 family members, and therapeutics known as IL-1 traps. IL-1ra polypeptides include the forms of IL-1ra described in US 5,075,222 and modified forms and variants including those described in U.S. 5,922,573, WO 91/17184, WO 92 16221, and WO 96 09323, all of which are. IL-1 beta conversing enzyme (ICE) inhibitors include peptidyl and small molecule ICE inhibitors including those described in PCT patent applications WO 91/15577; WO 93/05071; WO 93/09135; WO 93/14777 and WO 93/16710; and European patent application 0 547 699. Non-peptidyl compounds include those described in PCT patent application WO 95/26958. U.S. 5,552,400, U.S. 6,121,266:, Dolle et al., J. Med. Chem., 39, pp. 2438-2440 (1996). Additional ICE inhibitors are described in US Pat. Nos. 6,162,790, 6,204,261, 6,136,787, 6,103,711, 6,025,147, 6,008,217, 5,973,111, 5,874,424, 5,847,135, 5,843,904, 5,756,466, 5,656,627, 5,716,929.

IL-1 binding forms of type I IL-1 receptor and type II IL-1 receptor are described in U.S. 4,968,607, US 4,968.607, US 5,081,228, U.S. Re 35,450, U.S. 5,319,071, and 5,350,683. IL-1 traps are described in WO 018932.

Further, suitable IL-1 antagonists encompass chimeric proteins that include portions of both an antibody molecule and an IL-1 antagonist molecule. Such chimeric molecules may form monomers, dimers or higher order multimers. Other suitable IL-1 antagonists include peptides derived from IL-1 that are capable of binding competitively to the IL-1 signaling receptor, IL-1 R type I.

Preferred methods of the invention utilize type II IL-1 receptor in a form that binds IL-1 and particularly IL-1 beta, and blocks IL-1 signal transduction, thereby interrupting the proinflammatory and immunoregulatory effects of IL-1, and particularly that of IL-1 beta. U.S. Patent No. 5,350,683 describes type II IL-1 receptor polypeptide, The receptor polynucleotide sequence and the amino acid sequence that it encodes are provided herein as SEQ ID NO:1 and SEQ ID NO:2, respectively. Preferable forms of the type II IL-1 receptor polypeptide are truncated soluble fragments that retain the capability of binding IL-1 and particularly IL-1 beta. Soluble type II IL-1 receptor molecules include, for example, analogs or fragments of native type II IL-1 receptor having at least 20 amino acids, that lack the transmembrane region of the native molecule, and that are capable of binding IL-1, particularly IL-1 beta. A preferred soluble fragment of type II IL-1 receptor for use In the methods of the present invention includes amino acids 1-333 of SEQ ID NO:2. The preferred soluble type II IL-1 receptor is also the preferred IL-1 inhibitor for use in the methods of the present invention. It is recognized, however, that other inhibitors, including soluble forms of type I IL-1 receptor, IL-1ra, the foregoing mentioned antibodies, and derivative of IL-1 family members that bind cell bound receptors and inhibit signal transduction are useful in the practice of the present invention.

Antagonists derived from type II IL-1 receptors (e.g. soluble forms that bind IL-1 beta) compete for IL-1 with IL-1 receptors on the cell surface, thus inhibiting IL-1 from binding to cells, thereby preventing it from manifesting its biological activities. Binding of soluble type II IL-1 receptor or fragments of IL-1 or IL-1 beta can be assayed using ELISA or any other convenient assay. This invention additionally provides for the use of soluble forms of type II IL-1 receptor in the manufacture of a medicament for the treatment of numerous diseases. This invention additionally provides for the use of DNA encoding type II IL-1 receptor in the manufacture of soluble type II IL-1 receptor for use in the manufacture of a medicament for the treatment of diseases disclosed herein.

Soluble type II IL-1 receptor polypeptides or fragments suitable in the practice of this invention may be fused with a second polypeptide to form a chimeric protein. In one embodiment of such a chimeric protein, the second polypeptide may promote the spontaneous formation by the chimeric protein of a dimer, trimer or higher order multimer that is capable of binding IL-1 molecule and preventing it from binding to a cell-bound receptor that promotes IL-1 signaling. Chimeric proteins used as antagonists may be proteins that contain portions of both an antibody molecule and a soluble type II IL-1. receptor. A preferred IL-1 antagonist suitable for treating diseases in humans and other mammals is recombinant type II IL-1 receptors having amino acids 1-333 of SEQ ID NO:2

In one preferred embodiment of the invention, sustained-release forms of soluble 1L-1 receptors, and in particular, soluble type II IL-1 receptor are used. Sustained-release forms suitable for use in the disclosed methods include, but are not limited to, IL-1 receptors that are encapsulated in a slowly-dissolving biocompatible polymer, admixed with such a polymer, and or encased in a biocompatible semi-permeable implant. In addition, the soluble IL-1 receptor may be conjugated with polyethylene glycol (pegylated) to prolong its serum half-life or to enhance protein delivery. Soluble forms of IL-1 receptors, including monomers, fusion proteins (also called "chimeric proteins), dimers, trimers or higher order multimers, are particularly useful in formulating IL-1 antagonists.

To treat a medical disorder characterized by abnormal or excess expression of IL-1 or abnormal or excess IL-1 signaling, a molecule comprising an IL-binding soluble IL-1 receptor, preferably a soluble type II IL-1 receptor, is administered to the patient in an amount and for a time sufficient to induce a sustained improvement in at least one indicator that reflects the severity of the disorder. An improvement is considered "sustained" if the patient exhibits the improvement on at least two occasions separated by one to four weeks. The degree of improvement is determined based on signs or symptoms, and may also employ questionnaires that are administered to the patient, such as quality-of-life questionnaires.

Various indicators that reflect the extent of the patient's illness may be assessed for determining whether the amount and time of the treatment is sufficient. The baseline value for the chosen indicator or indicators is established by examination of the patient prior to administration of the first dose of the soluble type If IL-1 receptor or other IL-1 inhibitor. Preferably, the baseline examination is done within about 60 days of administering the first dose. If the IL-1 antagonist is being administered to treat acute symptoms, such as, for example, to treat traumatic injuries (traumatic knee injury, stroke, head injury, etc.) the first dose is administered as soon as practically possible after the injury or event has occurred,

Improvement is induced by repeatedly administering a dose of soluble type II IL-1 receptors or other IL-1 antagonist until the patient manifests an improvement over baseline for the chosen indicator or indicators. In treating chronic conditions, this degree of improvement is obtained by repeatedly administering this medicament over a period of at least a month or more, e.g., for one, two, or three months or longer, or indefinitely. A period of one to six weeks, or even a single dose, often is sufficient for treating acute conditions.

Although the extent of the patient's illness after treatment may appear improved according to one or more indicators, treatment may be continued indefinitely at the same level or at a reduced dose or frequency. Once treatment has been reduced or discontinued, it later may be resumed at the original level if symptoms should reappear.

Any efficacious route of administration may be used to therapeutically administer a soluble type II IL-1 receptor or other IL-1 antagonists. If injected, a soluble type II IL-1 receptor can be administered, for example, via intra-articular, intravenous, intramuscular, intralesional, intraperitoneal, intracranial, inhalation or subcutaneous routes by bolus injection or by continuous infusion. For example, pulmonary diseases can involve intranasal and inhalation methods. Other suitable means of administration include sustained release from implants, aerosol inhalation, eyedrops, oral preparations, including pills, syrups, lozenges or chewing gum, and topical preparations such as lotions, gels, sprays, ointments or other suitable techniques. Administration by inhalation is particularly beneficial when treating diseases associated with pulmonary disorders. Alternatively, IL-1 inhibitor polypeptides, such as a soluble IL-1 receptor, may be administered by implanting cultured cells that express the protein; for example, by implanting cells that express a soluble type II IL-1 receptor. In one embodiment, the patient's own cells are induced to produce by transfection in vivo or ex vivo with a DNA that encodes an IL-1 inhibitor or IL-1 antagonist, and particularly soluble type II IL-1 receptor. This DNA can be introduced into the patient's cells, for example, by injecting naked DNA or liposome-encapsulated DNA that encodes soluble type II IL-1 receptor or selected IL-1 antagonist, or by other means of transfection. When soluble type II IL-1 receptor is administered in combination with one or more other biologically active compounds, these may be administered by the same or by different routes, and may be administered simultaneously, separately or sequentially.

IL-1 inhibitors used in the methods of this invention, e.g. soluble type II IL-1 receptor or other soluble IL-1 receptors that are antagonists of IL-1, preferably are administered in the form of a physiologically acceptable composition comprising purified recombinant protein in conjunction with physiologically acceptable carriers, excipients or diluents. Such carriers are nontoxic to recipients at the dosages and concentrations employed. Ordinarily, preparing such compositions entails combining the IL-1 antagonist with buffers, antioxidants such as ascorbic acid, low molecular weight polypeptides (such as those having fewer than 10 amino acids), proteins, amino acids, carbohydrates such as glucose, sucrose or dextrins, chelating agents such as EDTA, glutathione and other stabilizers and excipients. Neutral buffered saline or saline mixed with conspecific serum albumin are exemplary appropriate diluents. The type II IL-1 receptor preferably is formulated as a lyophilizate using appropriate excipient solutions (e.g., sucrose) as diluents. Appropriate dosages can be determined in standard dosing trials, and may vary according to the chosen route of administration. In accordance with appropriate industry standards, preservatives may also be added, such as benzyl alcohol. The amount and frequency of administration will depend, of course, on such factors as the nature and severity of the indication being treated, the desired response, the age and condition of the patient, and so forth.

In one embodiment of the invention, type II IL-1 receptor is administered one time per week to treat the various medical disorders disclosed herein, in another embodiment, is administered at least two times per week, and in another embodiment is administered at least once per day. An adult patient is a person who is 18 years of age or older. If injected, the effective amount, per adult dose, ranges from 1-200 mg/m², or from 1-40 mg/m² or about 5-25 mg/m². Alternatively, a flat dose may be administered, whose amount may range from 2-400 mg/dose, 2-100 mg/dose or from about 10-80 mg/dose. If the dose is to be administered more than one time per week, an exemplary dose range is the same as the foregoing described dose ranges or lower. Preferably, type II IL-1R is administered two or more times per week at a per dose range of 25-100 mg/dose. In one embodiment of the invention, the various indications described below are treated by administering a preparation acceptable for injection containing type II IL-1 receptor at 80-100 mg/dose, or alternatively, containing 80 mg per dose. The dose is administered repeatedly. If a route of administration other than injection is used, the dose is appropriately adjusted in accord with standard medical practices. For example, if the route of administration is inhalation, dosing may be one to seven times per week at dose ranges from 10 mg/dose to 50 mg per dose.

In many instances, an improvement in a patents condition will be obtained by injecting a dose of up to about 100 mg of type II IL-1 receptor one to three times per week over a period of at least three weeks, though treatment for longer periods may be necessary to induce the desired degree of improvement. For incurable chronic conditions, the regimen may be continued indefinitely.

For pediatric patients (age 4-17), a suitable regimen involves the subcutaneous injection of 0.4 mg/kg to 5 mg/kg of type II IL-1 receptor, administered by subcutaneous injection one or more times per week.

The invention further includes the administration of type II IL-1 receptor concurrently with one or more other drugs that are administered to the same patient, each drug being administered according to a regimen suitable for that medicament. This encompasses pre-treatment, simultaneous treatment, sequential treatment and alternating regimens. Examples of such drugs include but are not limited to antivirals, antibiotics, analgesics, corticosteroids, antagonists of inflammatory cytokines, DMARDs and non-steroidal anti-inflammatories. Additionally, type II IL-1 receptor may be combined with a second IL-1 antagonist, including an antibody against IL-1 or an IL-1 receptor, additional IL-1 receptor derivatives, or other molecules that reduce endogenous IL-1 levels, such as inhibitors of the IL-1 beta converting enzyme and peptidomimetic IL-1 antagonists. In further embodiments, type II IL-1 receptor is administered in combination with pentoxifylline or thalidomide.

In an embodiment of the invention, the various medical disorders disclosed herein as being treatable with IL-1 inhibitors including soluble type II IL-1 receptor are treated in combination with another cytokine or cytokine inhibitor. For example, type II IL-1 receptor may be administered in a composition that also contains a compound that inhibits the interaction of other inflammatory cytokines with their receptors. The type II IL-1 receptor and other cytokine inhibitors may be administered as separate compositions, and these may be administered by the same or different routes. Examples of cytokine inhibitors used in combination with type II IL-1 receptor include those that antagonize, for example, TGFβ, IFNγ, IL-6 or IL-8 and TNF, particularly TNFα, The combination of an IL-1 inhibitor, e.g. type II IL-1R and IL-6 can be used to treat and prevent the recurrence of seizures, including seizures induced by GABA_{A} receptor antagonism, seizures associated with EEG ictal episodes and motor limbic seizures occurring during status epilepticus. Further, the combination of type II IL-1 receptor and IPNγ-1b is useful in treating idiopathic pulmonary fibrosis and cystic fibrosis. Other combinations for treating the herein described diseases include the use of type II IL-1 receptor with compounds that interfere with the binding of RANK and RANK-ligand, such as RANK-ligand inhibitors, or soluble forms of RANK, including RANK:Fc. For example, the combination of type II IL-1 receptor and RANK:Fc are useful for preventing bone destruction in various settings including but not limited to various rheumatic disorders, osteoporosis, multiple myeloma or other malignancies that cause bone degeneration, or anti-tumor therapy aimed at preventing metastasis to bone, or bone destruction associated with prosthesis wear debris or with periodontitis. IL-1 inhibitors such as type II IL-1 receptor also may be administered in combination with G-CSF, GM-CSF, IL-2 and inhibitors of protein kinase A type 1 to enhance T cell proliferation in HIV-infected patients who are receiving antiretroviral therapy. In addition, type II IL-1 receptor may be administered in combination with soluble forms of an IL-17 receptor (such as IL-17R:Fc), IL-18 binding protein, soluble forms of IL-18 receptors, and IL-18 antibodies, antibodies against IL-18 receptors or antibodies against CD30-ligand or against CD4.

Importantly, the present invention further encompasses methods for treating the herein disclosed medical disorders with a combination of an IL-1 inhibitor, preferably soluble type II IL-1 receptor (amino acids 1-333 of SEQ ID NO:2), a TNF inhibitor, preferably TNFR:Fc (ENBREL marketed for clinical uses by Immunex Corp) and any combination of the above described cytokines or cytokine inhibitors that are active agents in combination therapies. For example, in accordance with the present invention, combination therapy methods for treating rheumatoid arthritis, stroke, and congestive heart failure, include administering type II IL-1 receptor and ENBREL. Thus, the present invention also relates to the using IL-1 inhibitors and TNF inhibitors in combination therapies for use in medicine and in particular in therapeutic and preventive therapies for the medical disorders described herein. The use in medicine may involve the treatment of any of the medical disorders as described herein with a combination therapy that includes administering a combination of type II IL-1R and ENBREL. The IL-1 inhibitors (e.g. type II IL-1 receptor) and TNF inhibitor (ENBREL) may be in the form of compounds, compositions or combination therapies. Where the compounds are used together with one or more other components, the compound and the one or more other components may be administered simultaneously, separately or sequentially (usually in pharmaceutical format).

In connection with combination therapies of the present invention and in addition to ENBREL, other TNF antagonists include peptide fragments of TNFα, antisense oligonucleotides or ribozymes that inhibit TNFα production, antibodies directed against TNF (i.e. REMICADE), and recombinant proteins comprising all or portions of receptors for TNFα or modified variants thereof, including genetically-modified muteins, multimeric forms and sustained-release formulations. TNFα inhibitors disclosed in U.S. 5,641,751 and U.S. 5,519,000, and the D-amino acid-containing peptides described in U.S. 5,753,628 and inhibitors of TNFα converting enzyme.

Other compounds that are TNF inhibitors that may be used in combination therapies include small molecules such as thalidomide or thalidomide analogs, pentoxifylline, or matrix metalloproteinase (MMP) inhibitors or other small molecules. Suitable MMP inhibitors include, for example, those described in U.S. Patent Nos. 5,883,131, 5,863,949 and 5,861,510 as well as the mercapto alkyl peptidyl compounds described in U.S. 5,872,146. Other small molecules capable of reducing TNFα production, include, for example, the molecules described in U.S. Patent Nos. 5,508,300, 5,596,013 and 5,563,143, any of which can be administered in combination with TNFα inhibitors such as soluble TNFRs or antibodies against TNFα. Additional small molecules useful for treating the TNFα-mediated diseases described herein include the MMP inhibitors that are described in U.S. 5,747,514, U.S. 5,691,382, as well as the hydroxamic acid derivatives described in U.S. 5,821,262. The diseases described herein also may be treated with small molecules that inhibit phosphodiesterase IV and TNFα production, such as substituted oxime derivatives (WO 96/00215), quinoline sulfonamides (U.S. 5,834,485), aryl furan derivatives (WO 99/18095) and heterobicyclic derivatives (WO 96/01825; GB 2 291422 A). Also useful are thiazole derivatives that suppress TNFα and IFNγ (WO 99/15524), as well as xanthine derivatives that suppress TNFα and other proinflammatoty cytokines (see, for example, U.S. 5,118,500, U.S. 5,096,906 and U.S. 5,196,430). Additional small molecules useful for treating the hereindescribed conditions include those disclosed in U.S. 5,547,979.

Antisense oligonucleotides for suitable for treating diseases in combinations include, for example, the anti-TNFα oligonucleotides described in U.S. Patent No. 6,080,580, which proposes the use of such oligonucleotides as candidates for testing in animal models of diabetes mellitus, rheumatoid arthritis, contact sensitivity, Crohn's disease, multiple sclerosis, pancreatitis, hepatitis and heart transplant.

Preferred embodiments of the combination therapies described herein utilize soluble TNFRs as the TNFα antagonist. Soluble forms of TNFRs may include monomers, fusion proteins (also called "chimeric proteins), dimers, trimers or higher order multimers. In certain embodiments of the invention, the soluble TNFR derivative is one that mimics the 75 kDa TNFR or the 55 kDa TNFR and that binds to TNFα in the patient's body. The soluble TNFR mimics of the present invention may be derived from TNFRs p55 or p75 or fragments thereof. TNFRs other than p55 and p75 also are useful for deriving soluble compounds for treating the various medical disorders described herein, such for example the TNFR that is described in WO 99/04001. Soluble TNFR molecules used to construct TNFR mimics include, for example, analogs or fragments of native TNFRs having at least 20 amino acids, that lack the transmembrane region of the native TNFR, and that are capable of binding TNFα. Antagonists derived from TNFRs compete for TNFα with the receptors on the cell surface, thus inhibiting TNTα from binding to cells, thereby preventing it from manifesting its biological activities. Binding of soluble TNFRs to TNFα or LTα can be assayed using ELISA or any other convenient assay. This invention provides for the use of soluble TNFα receptors in the manufacture of medicaments for the treatment of numerous diseases.

In addition, the subject invention provides methods for treating a human patient in need thereof, the method involving administering to the patient a therapeutically effective amount of an IL-1 inhibitor, including the aforementioned IL-1 inhibitors, an IL-4 inhibitor, and optionally, a TNFα inhibitor, e,g. ENBREL, and any of the aforementioned combination therapies.

IL-4 antagonists that may be employed in accordance with the present invention include, but are not limited to, IL-4 receptors (IL-4R) and other IL-4-binding molecules, IL-4 muteins and antibodies that bind specifically with IL-4 or IL-4 receptors thereby blocking signal transduction, as well as antisense oligonucleotides and ribozymes targeted to IL-4 or IL-4R. Antibodies specific for IL-4 or IL-4 receptor may be prepared using standard procedures. Among the IL-4 receptors suitable for use as described herein are soluble fragments of human IL-4R that retrain the ability to bind IL-4. Such fragments are capable of binding IL-4, and retain all or part of the IL-4R extracellular region.

IL-4 antagonists useful for the hereindescribed combination methods of treatment include molecules that selectively block the synthesis of endogenous IL-4 or IL-4R. IL-4 receptors are described in U.S. Patent 5,599,905; Idzerda et al., J. Exp. Med. 171:861-873, March 1990 (human IL-4R); and Mosley et al., Cell 59:335-348, 1989 (murine IL-4R), each of which is hereby incorporated by reference in its entirety. The protein described in those three references is sometimes referred to in the scientific literature as IL-4Rα. Unless otherwise specified, the terms "IL-4R" and "IL-4 receptor" as used herein encompass this protein in various forms that are capable of functioning as IL-4 antagonists, including but not limited to soluble fragments, fusion proteins, oligomers, and variants that are capable of binding IL-4, as described in more detail below. Suitable IL-4Rs include variants in which valine replaces isoleucine at position 50 (see Idzerda et al., 1990), and include slow-release formulations, and PEGylated derivatives (modified with polyethylene glycol) are contemplated, as well as recombinant fusion proteins comprising heterologous polypeptides fused to the N-terminus or C-terminus of an IL-4R polypeptide, including signal peptides, immunoglobulin Fc regions, poly-His tags or the FLAG^{®} polypeptide described in Hopp et al., Bio/Technology 6:1204, 1988, and U.S. Patent 5,011,912, as well as fusions of IL-4 receptors with oligomer-promoting leucine zipper moieties. Soluble recombinant fusion proteins comprising an IL-4R and immunoglobulin constant regions are described, for example, in EP 464,533.

Various IL-4 antagonists that may be used for the hereindescribed methods of treatment can be identified, for example, by their ability to inhibit ³H-thymidine incorporation in cells that normally proliferate in response to IL-4, or by their ability to inhibit binding of IL-4 to cells that express IL-4R. In one assay for detecting IL-4 antagonists, one measures the ability of a putative antagonist to block the IL-4-induced enhancement of the expression of CD23 on the surfaces of human B cells. For example, B cells isolated from human peripheral blood are incubated in microtiter wells in the presence of IL-4 and the putative antagonist. Following the incubation, washed cells are then incubated with labeled monoclonal antibody against CD23 (available from Pharmingen) to determine the level of CD23 expression. An anti-huIL-4R murine mAb (R&D Systems), previously shown to block the binding and function of both hIL-4 and hIL-13, may used as a positive control for neutralization of CD23 induction by IL-4. Alternatively, suitable IL-4 antagonists may be identified by determining their ability to prevent or reduce the impaired the barrier function of epithelium that results when IL-4 is incubated with the epithelium. For this purpose, one may use confluent monolayers of human epithelial cell lines such as Calu-3 (lung) or T84 (intestinal epithelium). Incubation of such monolayers with IL-4 causes significant damage to their barrier function within about 48 hours. To assay IL-4 antagonists, monolayers may be tested for their permeability, for example, by adding radiolabeled mannitol to cells incubated with IL-4 in the presence or absence of an antagonist. Alternatively, transepithelial resistance (indicating an intact barrier) may be determined using a voltmeter.

Combinations of one of more IL-1 inhibitors and IL-4 inhibitors, and optionally TNFα inhibitors, e.g. ENBREL, preferably are administered one or more times per week. The mode of administration of IL-4 inhibitors and IL-1 inhibitors can depend upon the medical condition treated and include modes described above including subcutaneous injection and by inhalation nasally. Suitable dose ranges for IL-4 antagonists include doses of from about 1 ng/kg/day to about 10 mg/kg/day, more preferably from about 500 ng/kg/day to about 5 mg/kg/day, and most preferably from about 5 µg/kg/day to about 2 mg/kg/day, administered to adults one time per week, two times per week, or three or more times per week. If injected, suitable doses may range from 1-20 mg/m², and preferably is about 5-12 mg/m². Alternatively, a flat dose of about 5-100 mg/dose may be used, preferably about 20-30 mg per dose. For pediatric patients (age 4-17), one suitable regimen involves subcutaneous injection of 0.4 mg/kg, up to a maximum dose of 25 mg of IL-4R, administered two or three times per week. Another embodiment is directed to aerosol pulmonary administration, for example by nebulizer, which optimally will deliver a dose of 3 or more mg of a soluble IL-4R, and is taken at least once a week. Aeresolized IL-4R may be administered orally or nasally. One illustrative embodiment involves subcutaneous injection of a soluble human IL-4R once a week, at a dose of 1.5 to 3 mg. Doses will be adjusted as needed by the patient's physician in accord with standard medical practices.

Conditions effectively treated by a combination of an IL-1 inhibitor and an IL-4 inhibitor include conditions in which IL-1 and IL-4 play a role in the inflammatory response. Lung disorders in which IL-4 plays a role include asthma, chronic obstructive pulmonary disease, pulmonary alveolar proteinosis, bleomycin-induced pneumopathy and fibrosis, radiation-induced pulmonary fibrosis, cystic fibrosis, collagen accumulation in the lungs, and ARDS, all of which may be treated with combinations of an IL-1 inhibitor and an IL-4 inhibitor. Combinations of IL-1 inhibitors and IL-4 inhibitors also are useful for treating patients suffering from various skin disorders, including but not limited to dermatitis herpetiformis (Duhring's disease), atopic dermatitis, contact dermatitis, urticaria (including chronic idiopathic urticaria), and autoimmune blistering diseases, including pemphigus vulgaris and bullous pemphigoid. Other diseases treatable with the combination of an IL-1 inhibitor and an IL-4 inhibitor include myesthenia gravis, sarcoidosis, including pulmonary sarcoidosis, scleroderma, reactive arthritis, hyper IgE syndrome, multiple sclerosis and idiopathic hypereosinophil syndrome. The combination is used also for treating allergic reactions to medication and as an adjuvant to allergy immunotherapy. In connection with combination therapies, the combination of IL-1 inhibitor and IL-4 inhibitor, e.g. soluble type II IL-1R and soluble IL-4R, the aforementioned combination methods can further include the administration of TNFα inhibitors.

The present invention also relates to the use of IL-1 inhibitors (as disclosed), such as soluble type II IL-1 receptor, in the manufacture of a medicament for the prevention or therapeutic treatment of each medical disorder disclosed herein.

The disclosed IL-1 inhibitors, compositions and combination therapies described herein are useful in medicines for treating and/or preventing bacterial, viral or protozoal infections, and complications resulting therefrom. One such disease is *Mycoplasma pneumonia.* In addition, provided herein is the use of soluble type II IL-1 receptor compositions or combinations, particularly in combination with ENBREL to treat AIDS and conditions associated with AIDS and/or related to AIDS, such as AIDS dementia complex, AIDS associated wasting, lipidistrophy due to antiretroviral therapy; CMV (cytomegalovirus) and Kaposi's sarcoma. Furthermore provided herein is the use of soluble type II IL-1 receptor compositions or combinations for treating protozoal diseases, including malaria and schistosomiasis. Additionally provided is the use of IL-1 inhibitor such as soluble type II IL-1 receptor to treat erythema nodosum leprosum; bacterial or viral meningitis; tuberculosis, including pulmonary tuberculosis; and pneumonitis secondary to a bacterial or viral infection. Provided also herein is the use of soluble type II IL-1 receptor compositions or combinations to prepare medicaments for treating louse-borne relapsing fevers, such as that caused by *Borrelia recurrentis*. Soluble type II IL-1 receptor can also be used to prepare a medicament for treating conditions caused by *Herpes* viruses, such as herpetic stromal keratitis, corneal lesions; and virus-induced corneal disorders. In addition, soluble type II IL-1 receptor compositions and combinations can be used in treating human papillomavirus infections. Soluble type II IL-1 receptor is used also to prepare medicaments to treat influenza infection and infectious mononucleosis.

Cardiovascular disorders and injuries are treatable and/or preventable with the disclosed IL-1 inhibitors, pharmaceutical compositions or combination therapies. In particularly cardiovascular disorders are treatable with soluble type II IL-1 receptor compositions, alone or in combination with TNF inhibitors (e.g. ENBREL) and or other agents as described above. Cardiovasuclar disorders thus treatable include aortic aneurysms; including abdominal aortic aneurysms, acute coronary syndrome, arteritis; vascular occlusion, including cerebral artery occlusion; complications of coronary by-pass surgery; ischemia/reperfusion injury; heart disease, including atherosclerotic heart disease, myocarditis, including chronic autoimmune myocarditis and viral myocarditis; heart failure, including chronic heart failure, congestive heart failure, cachexia of heart failure; myocardial infarction; restenosis and/or atherosclerosis after heart surgery or after carotid artery balloon angioplastic procedures; silent myocardial ischemia; left ventricular pump dysfunction, post implantation complications of left ventricular assist devices; Raynaud's phenomena; thrombophlebitis; vasculitis, including Kawasaki's vasculitis; veno-occlusive disease, giant cell arteritis, Wegener's granulomatosis; mental confusion following cardio pulmonary by pass surgery, and Schoenlein-Henoch purpura. Combinations of IL-1-inhibitors, TNT inhibitors and angiogenesis inhibitors (e.g. anti-VEGF) are useful for treating certain cardiovascular diseases such as aortic aneurysms and tumors.

In addition, the subject IL-1 inhibitors, compositions and combination therapies are used to treat chronic pain conditions, such as chronic pelvic pain, including chronic prostatitis/pelvic pain syndrome. As a further example, soluble type II IL-1 receptor and the compositions and combination therapies of the invention are used to treat post-herpetic pain.

Provided also are methods for using IL-1 inhibitors, compositions or combination therapies to treat various disorders of the endocrine system. For example, type II IL-1 receptor compositions or other IL-1 inhibitor compositions, with or without TNF inhibitors (ENBREL) or other active agents described above, are suitable for use to treat juvenile onset diabetes (includes autoimmune diabetes mellitus and insulin-dependent types of diabetes) and also to treat maturity onset diabetes (includes non-insulin dependent and obesity-mediated diabetes). In addition, the subject compounds, compositions and combination therapies are used to treat secondary conditions associated with diabetes, such as diabetic retinopathy, kidney transplant rejection in diabetic patients, obesity-mediated insulin resistance, and renal failure, which itself may be associated with proteinurea and hypertension. Other endocrine disorders also are treatable with these compounds, compositions or combination therapies, including polycystic ovarian disease, X-Iinked adrenoleukodystrophy, hypothyroidism and thyroiditis, including Hashimoto's thyroiditis (i.e., autoimmune thyroiditis). Further, IL-1 inhibitors, including type II IL-1 receptor, alone or in combination with other cytokines, including TNF inhibitors such as ENBREL, are useful in treating or preventing medical conditions associated with thyroid cell dysfunction, including euthyroid sick syndrome.

Conditions of the gastrointestinal system are treatable or preventable with IL-1 inhibitors, compositions or combination therapies, including coeliac disease, For example, type II IL-1 receptor compositions, with or without TNF inhibitors (ENBREL) or other active agents described above are suitable for treating or preventing coeliac disease. In addition, the compounds, compositions and combination therapies of the invention are suitable for treating or preventing Crohn's disease; ulcerative colitis; idiopathic gastroparesis; pancreatitis, including chronic pancreatitis; acute pancreatitis, inflammatory bowel disease and ulcers, including gastric and duodenal ulcers.

Included also are methods for using the subject IL-1 inhibitors, compositions or combination therapies for treating disorders of the genitourinary system. For example, type II IL-1 receptor compositions, alone or in combination with TNF inhibitors (ENBREL) or other active agents described above are suitable for treating or preventing glomerulonephritis, including autoimmune glomerulonephritis, glomerulonephritis due to exposure to toxins or glomerulonephritis secondary to infections with haemolytic streptococci or other infectious agents. Also treatable with the compounds, compositions and combination therapies of the invention are uremic syndrome and its clinical complications (for example, renal failure, anemia, and hypertrophic cardiomyopathy), including uremic syndrome associated with exposure to environmental toxins, drugs or other causes. IL-1 inhibitors, particularly type II IL-1 receptor, alone or in combination with TNF inhibitors, particularly ENBREL, are useful in treating and preventing complications that arise from inflammation of the gallbladder wall that leads to alteration in absorptive function. Included in such complications are cholelithiasis (gallstones) and choliedocholithiasis (bile duct stones) and the recurrence of cholelithiasis and choliedochohthiasis. Further conditions treatable with the compounds, compositions and combination therapies of the invention are complications of hemodialysis; prostate conditions, including benign prostatic hypertrophy, nonbacterial prostatitis and chronic prostatitis; and complications of hemodialysis.

Also provided herein are methods for using IL-1 inhibitors, compositions or combination therapies to treat various hematologic and oncologic disorders. For example, soluble type II IL-1 receptor, alone or in combination with a TNF inhibitor (ENBREL) or other active agents as described above, may be used to treat, various forms of cancer, including acute myelogenous leukemia, chronic myelogenous leukemia leukemia, Epstein-Barr virus-positive nasopharyngeal carcinoma, glioma, colon, stomach, prostate, renal cell, cervical and ovarian cancers, lung cancer (SCLC and NSCLC), including cancer-associated cachexia, fatigue, asthenia, paraneoplastic syndrome of cachexia and hypercalcemia. Additional diseases treatable with the subject IL-1 inhibitors, compositions or combination therapies are solid tumors, including sarcoma, osteosarcoma, and carcinoma, such as adenocarcinoma (for example, breast cancer) and squamous cell carcinoma. In addition, the subject compounds, compositions or combination therapies are useful for treating esophogeal cancer, gastric cancer, gall bladder carcinoma, leukemia, including acute myelogenous leukemia, chronic myelogenous leukemia, myeloid leukemia, chronic or acute lymphoblastic leukemia and hairy cell leukemia. Other malignancies with invasive metastatic potential, including multiple myeloma, can be treated with the subject compounds, compositions and combination therapies, and particularly combination therapies that include soluble type II IL-1 receptor and soluble TNF receptor (ENBREL). In addition, the disclosed IL-1 inhibitors, compositions and combination therapies can be used to treat anemias and hematologic disorders, including chronic idiopathic neutropenia, anemia of chronic disease, aplastic anemia, including Fanconi's aplastic anemia; idiopathic thrombocytopenic purpura (ITP); thrombotic thrombocytopenic purpura, myelodysplastic syndromes (including refractory anemia, refractory anemia with ringed sideroblasts, refractory anemia with excess blasts, refractory anemia with excess blasts in transformation); myelofibrosis/myeloid metaplasia; and sickle cell vasocclusive crisis.

Various lymphoproliferative disorders also are treatable with the disclosed IL-1 inhibitors, compositions or combination therapies. Type II IL-1 receptor, alone or in combination with a TNF inhibitor, such as ENBREL, or other active agents are useful for treating or preventing autoimmune lymphoproliferative syndrome (ALPS), chronic lymphoblastic leukemia, hairy cell leukemia, chronic lymphatic leukemia, peripheral T-cell lymphoma, small lymphocytic lymphoma, mantle cell lymphoma, follicular lymphoma, Burkitt's lymphoma, Epstein-Barr virus-positive T cell lymphoma, histiocytic lymphoma, Hodgkin's disease, diffuse aggressive lymphoma, acute lymphatic leukemias, T gamma lymphoproliferative disease, cutaneous B cell lymphoma, cutaneous T cell lymphoma (i.e., mycosis fungoides) and Sézary syndrome.

In addition, the subject IL-1 inhibitors, compositions and combination therapies are used to treat hereditary conditions. In particular, type II IL-1 receptor, alone or in combination with a TNF inhibitor such as ENBREL, is useful to treat diseases such as Gaucher's disease, Huntington's disease, linear IgA disease, and muscular dystrophy.

Other conditions treatable or preventable by the disclosed IL-1 inhibitors, compositions and combination therapies include those resulting from injuries to the head or spinal cord including subdural hematoma due to trauma to the head. For example, soluble type II IL-1 receptor, alone or in combination with a TNF inhibitor such as ENBREL are useful in treating head injuries and spinal chord injuries. In connection with this therapy, the compositions and combinations described are suitable for preventing cranial neurologic damage and preventing and treating cervicogenic headache. The compositions and combinations described are further suitable for treating neurological side effects associated with brain irradiation.

The disclosed IL-1 inhibitors, compositions and combination therapies are further used to treat conditions of the liver. For example soluble type II IL-1 receptor, alone or in combination with a TNF inhibitor such as ENBREL or other active agents, can be used to treat hepatitis, including acute alcoholic hepatitis, acute drug-induced or viral hepatitis, hepatitis A, B and C, sclerosing cholangitis and inflammation of the liver due to unknown causes. In connection with liver inflammation, IL-1 inhibitors are further useful in treating hepatic sinusoid epithelium

In addition, the disclosed IL-1 inhibitors, compositions and combination therapies are used to treat various disorders that involve hearing loss and that are associated with abnormal IL-1 expression. For example, soluble type II IL-1 receptor, alone or in combination with TNF inhibitors, can be used to treat or prevent cochlear nerve-associated hearing loss that is thought to result from an autoimmune process, i,e., autoimmune hearing loss. This condition currently is treated with steroids, methotrexate and/or cyclophosphamide. Also treatable or preventable with the disclosed IL-1 inhibitors, compositions and combination therapies is Meniere's syndrome and cholesteatoma, a middle car disorder often associated with hearing loss.

In addition, the subject invention provides IL-1 inhibitors, e.g. soluble type II IL-1 receptor, compositions and combination therapies (e.g. soluble type II IL-1 receptor and a TNF inhibitor such as ENBREL or other active agents) for the treatment of non-arthritic medical conditions of the bones and joints. This encompasses osteoclast disorders that lead to bone loss, such as but not limited to osteoporosis, including post-menopausal osteoporosis, osteoarthritis, periodontitis resulting in tooth loosening or loss, and prosthesis loosening after joint replacement (generally associated with an inflammatory response to wear debris). This latter condition also is called "orthopedic implant osteolysis." Another condition treatable with the compounds, compositions and combination therapies of the invention is temporal mandibular joint dysfunction (TMJ).

The following pulmonary disorders also can be treated or prevented with the disclosed IL-1 inhibitors, in particular soluble type II IL-1 receptor, compositions and combination therapies (e.g. type II IL-1 receptor and a TNF inhibitor such as ENBREL or other active agents): adult respiratory distress syndrome (ARDS), acute respiratory distress syndrome and acute lung injury caused by a variety of conditions, including exposure to toxic chemicals, pancreatitis, trauma or other causes of inflammation. The disclosed compounds, compositions and combination therapies of the invention also are useful for treating broncho-pulmonary dysplasia (BPD); chronic obstructive pulmonary diseases (e.g. emphysema and chronic bronchitis), and chronic fibrotic lung disease of preterm infants. In addition, the compounds, compositions and combination therapies of the invention are used to treat occupational lung diseases, including asbestosis, coal worker's pneumoconiosis, silicosis or similar conditions associated with long-term exposure to fine particles. In other aspects of the invention, the disclosed compounds, compositions and combination therapies are used to treat bronchioliterans organizing pneumonia, pulmonary fibrosis, including idiopathic pulmonary fibrosis and radiation-induced pulmonary fibrosis; pulmonary sarcoidosis; and allergies, including allergic rhinitis, contact dermatitis, atopic dermatitis and asthma.

Other embodiments of the present invention include methods for using the disclosed IL-1 inhibitors, in particular soluble type II IL-1 receptor, compositions or combination therapies, e.g. soluble type II IL-1 receptor and ENBREL, to treat or prevent a variety of rheumatic disorders. These include adult and juvenile rheumatoid arthritis; scleroderma; systemic lupus erythematosus: gout; osteoarthritis; polymyalgia rheumatica; seronegative spondylarthropathies, including ankylosing spondylitis, and Reiter's disease. The subject IL-1. inhibitors, compositions and combination therapies are used also to treat psoriatic arthritis and chronic Lyme arthritis. Also treatable or preventable with these compounds, compositions and combination therapies are Still's disease and uveitis associated with rheumatoid arthritis. In addition, the compounds, compositions and combination therapies of the invention are used in treating disorders resulting in inflammation of the voluntary muscle and other muscles, including dermatomyositis, inclusion body myositis, polymyositis, and lymphangioleimyomatosis.

The IL-1 inhibitors, e.g. soluble type II IL-1 receptor, compositions and combination therapies (e.g. an IL-1 inhibitor as soluble type II IL-1 receptor in combination with ENBREL or other TNF inhibitor or active agent) of the invention are useful for treating or preventing primary amyloidosis. In addition, the secondary amyloidosis that is characteristic of various conditions also are treatable with IL-1 inhibitors such as soluble type II IL-1 receptor, and the compositions and combination therapies described herein. Such conditions include: Alzheimer's disease, secondary reactive amyloidosis; Down's syndrome; and dialysis-associated amyloidosis. Also treatable with the compounds, compositions and combination therapies of the invention are inherited periodic fever syndromes, including familial Mediterranean fever, hyperimmunoglobulin D and periodic fever syndrome and TMF-receptor associated periodic syndromes (TRAPS).

Disorders involving the skin or mucous membranes also are treatable using the disclosed IL-1. inhibitors, such as soluble type II IL-1 receptor, compositions or combination therapies, e.g. soluble type II IL-1 receptor and ENBREL. Such disorders include acantholytic diseases, including Darier's disease, keratosis follicularis and pemphigus vulgaris. Also treatable with the subject IL-1 inhibitors, especially soluble type II IL-1 receptor, compositions and combination therapies are acne; acne rosacea; alopecia areata; aphthous stomatitis; bullous pemphigoid; burns; eczema; erythema, including erythema multiforme and erythema multiforme bullosum (Stevens-Johnson syndrome); inflammatory skin disease; lichen planus; linear IgA bullous disease (chronic bullous dermatosis of childhood); loss of skin elasticity; mucosal surface ulcers, including gastric ulcers; neutrophilic dermatitis (Sweet's syndrome); dermatomyositis, pityriasis rubra pilaris; psoriasis; pyoderma gangrenosum; multicentric reticulohistiocytosis; and toxic epidermal necrolysis. Other skin related conditions treatable by the therapies and combination therapies of the present invention include dermatitis herpetiformis

Disorders associated with transplantation also are treatable or preventable with the disclosed IL-1 inhibitors, such as soluble type If IL-1 receptor, compositions or combination therapies, including compositions of soluble type II IL-1 receptor and ENBREL. Such disorders include graft -versus-host disease, and complications resulting from solid organ transplantation, such as heart, liver, skin, kidney, lung (lung transplant airway obliteration) or other transplants, including bone marrow transplants.

Ocular disorders also are treatable or preventable with the disclosed IL-1 inhibitors, especially soluble type II IL-1 receptor, compositions or combination therapies, including rhegmatogenous retinal detachment, and inflammatory eye disease, including inflammatory eye disease associated with smoking and macular degeneration. IL-1 inhibitors such as soluble type II IL-1 receptor and the disclosed compositions and combination therapies also are useful for treating disorders that affect the female reproductive system. Examples include, but are not limited to, multiple implant failure/infertility; fetal loss syndrome or IV embryo loss (spontaneous abortion); preeclamptic pregnancies or eclampsia; endometriosis, chronic cervicitis, and pre-term labor.

In addition, the disclosed IL-1 inhibitors, particularly soluble type II IL-1 receptor compositions and combination therapies, such as combinations of type II preceptor and ENBREL are useful for treating obesity, including to bring about a decrease in leptin Also, the compounds, compositions and combination therapies of the invention are used to treat or prevent sciatica, symptoms of aging, severe drug reactions (for example, II-2 toxicity or bleomycin-induced pneumopathy and fibrosis), or to suppress the inflammatory response prior, during or after the transfusion of allogeneic red blood cells in cardiac or other surgery, or in treating a traumatic injury to a limb or joint, such as traumatic knee injury. Various other medical disorders treatable with the disclosed IL-1 inhibitors, compositions and combination therapies include; multiple sclerosis; Behcet's syndrome; Sjogren's syndrome; autoimmune hemolytic anemia; beta thalassemia; amyotrophic lateral sclerosis (Lou Gehrig's Disease); Parkinson's disease; and tenosynovitis of unknown cause, as well as various autoimmune disorders or diseases associated with hereditary deficiencies, including x-linked mental retardation.

The disclosed IL-1 inhibitors, particularly soluble type II IL-1 receptor, compositions and combination therapies, e.g. soluble type II IL-1 receptor and ENBREL, are useful for treating central nervous system (CNS) injuries, including the effects of neurotoxic neurotransmitters discharged during excitation of inflammation in the central nervous system and to inhibit or prevent the development of glial scars at sites of central nervous system injury. In connection with central nervous system medical conditions, IL-1 inhibitors, alone or in combination with TNF inhibitors and particularly type II IL-1 receptor and/or ENBREL are useful in treating temporal lobe epilepsy. In connection with epilepsy and the treatment of seizures, reducing the severity and number of recurring seizures, and reducing the severity of the deleterious effects of seizures. IL-1 inhibitors, in particular soluble type II IL-1R, alone or in combination with agents described herein, e.g. IL-6, is useful for reducing neuronal loss, neuronal degeneration, and gliosis associated with seizures.

Furthermore, the disclosed IL-1 inhibitors, particularly soluble type II IL-1, receptor, compositions and combination therapies, e.g. soluble type II IL-1 receptor and ENBREL, are useful for treating critical illness polyneuropathy and myopathy (CIPNM) acute polyneuropathy; anorexia nervosa; Bell's palsy; chronic fatigue syndrome; transmissible dementia, including Creutzfeld-Jacob disease; demyelinating neuropathy; Guillain-Barre syndrome; vertebral disc disease; Gulf war syndrome; chronic inflammatory demyelinating polyneuropathy, myasthenia gravis; silent cerebral ischemia; sleep disorders, including narcolepsy and sleep apnea; chronic neuronal degeneration; and stroke, including cerebral ischemic diseases. Other diseases and medical conditions that may be treated or prevented by administering an IL-1 inhibitor, such as soluble type II IL-1. receptor, alone or in combination with a herein described active agents, particularly a TNF inhibitor such as ENBREL, include anorexia and/or anorexic conditions, peritonitis, endotoxemia and septic shock, granuloma formation, heat stroke, Churg-Strauss syndrome, chronic inflammation following acute infections such as tuberculosis and leprosy, systemic sclerosis and hypertrophic scarring. In addition to IL-1 inhibitors in combination with TNF inhibitors, IFN-alpha beta or gamma and/or IL-4 inhibitors are suitable for treating hypertrophic scarring.

The IL-1 inhibitors discloses herein, and particularly soluble forms of type II IL-1R, IL-1ra and variants, and IL-1 traps, are useful for reducing the toxicity associated with antibody therapies, chemotherapy, radiation therapy and the effects of other apoptosis inducing agents, e.g. TRAIL and TRADE, and therapies that target IL-1 producing cells or illicit an inflammatory response. Monoclonal antibody therapies, chemotherapies and other apoptosis inducing therapies that target IL-1 producing cells induce the production and/or release of IL-1. By administering therapies that inhibit the effects of IL-1 by interfering with its interaction with its receptor and/or receptor accessory, the proinflammatory effects and medical conditions associated with IL-1 are reduced or eliminated.

In addition to human patients, soluble type II IL-1 receptors are useful in the treatment of non-human animals, such as pets (dogs, cats, birds, primates, etc.), domestic farm animals (horses cattle, sheep, pigs, birds, etc.), or any animal that suffers from an IL-1-mediated inflammatory or arthritic condition. In such instances, an appropriate dose may be determined according to the animal's body weight For example, a dose of 0.2-1 mg/kg may be used. Alternatively, the dose is determined according to the animal's surface area, an exemplary dose ranging from 0.1-20 mg/m², or more preferably, from 5-12 mg/m². For small animals, such as dogs or cats, a suitable dose is 0.4 mg/kg. Soluble type II IL-1 receptor (preferably constructed from genes derived from the recipient species), or another soluble IL-1 receptor mimic, is administered by injection or other suitable route one or more times per week until the animal's condition is improved, or it may be administered indefinitely.

Provided herein are methods of treating or preventing psoriatic lesions that involve administering to a human patient a therapeutically effective amount of a soluble IL-1 receptor. A preferred soluble for this purpose is soluble type II IL-1 receptor. The treatment is effective against psoriatic lesions that occur in patients who have ordinary psoriasis or psoriatic arthritis.

Patients are defined as having ordinary psoriasis if they lack the more serious symptoms of psoriatic arthritis (e.g., distal interphalangeal joint DIP involvement, enthesopathy, spondylitis and dactylitis), but exhibit one of the following: 1) inflamed swollen skin lesions covered with silvery white scale (plaque psoriasis or psoriasis vulgaris); 2) small red dots appearing on the trunk, arms or legs (guttate psoriasis); 3) smooth inflamed lesions without scaling in the flexural surfaces of the skin (inverse psoriasis); 4) widespread reddening and exfoliation of fine scales, with or without itching and swelling (erythrodermic psoriasis); 5) blister-like lesions (pustular psoriasis); 6) elevated inflamed scalp lesions covered by silvery white scales (scalp psoriasis); 7) pitted fingernails, with or without yellowish discoloration, crumbling nails, or inflammation and detachment of the nail from the nail bed (nail psoriasis).

In treating ordinary psoriasis, soluble type II IL-1 receptor is administered in an amount and for a time sufficient to induce an improvement in the patient's condition as measured according to any indicator that reflects the severity of the patient's psoriatic lesions. One or more such indicators may be assessed for determining whether the amount of IL-1 inhibitor and duration of treatment is sufficient. In one preferred embodiment of the invention, the soluble type II IL-1 receptor is administered in an amount and for a time sufficient to induce an improvement over baseline in either the psoriasis area and severity index (PASI) or the Target Lesion Assessment Score. In another embodiment, both indicators are used. When PASI score is used as the indicator, treatment is regarded as sufficient when the patient exhibits an at least 50% improvement in his or her PASI score, or alternatively, when the patient exhibits an at least 75% improvement in PASI score. Using the Psoriasis Target Lesion Assessment Score to measure sufficiency of treatment involves determining for an individual psoriatic lesion whether improvement has occurred in one or more of the following, each of which is separately scored: plaque elevation; amount and degree of scaling or degree of erythema; and target lesion response to treatment. Psoriasis Target Lesion Assessment Score is determined by adding together the separate scores for all four of the aforementioned indicia, and determining the extent of improvement by comparing the baseline score the score after treatment has been administered.

A satisfactory degree of improvement in psoriasis patients is obtained by administering the soluble type II IL-1 receptor one or more times per week. For example, the soluble type II IL-1 receptor may be administered one time, two times or three or more times per week. Treatment may be continued over a period of at least one week, for two weeks, three weeks, four weeks or longer. Treatment may be discontinued after the patient improves, then resumed if symptoms return, or alternatively, the treatment may be administered continuously for an indefinite period. A preferred route of administration is subcutaneous injection. In one preferred method for treating adult psoriasis patients, the amount of soluble type. II IL-1 receptor administered by injection is 5-12 mg/m², or a flat dose of either 25 mg or 50 mg. In one preferred embodiment of this method, a dose of 25 mag is injected two times per week, and in another preferred embodiment, a dose of 50 mg is injected one time per week. In a preferred method of treating pediatric psoriasis patients, the dose administered by injection is 0.4 mg/kg, up to a maximum dose of 25 mg.

Soluble type II IL-1 receptor may be used to treat ordinary psoriasis in combination with one, two, three or more other medications that are effective against psoriasis. These additional medications may be administered before, simultaneously with, or sequentially with the soluble type II IL-1 receptor. Drugs suitable for combination therapies of psoriasis include pain medications (analgesics), including but not limited to acetaminophen, codeine, propoxyphene napsylate, oxycodone hydrochloride, hydrocodone bitartrate and tramadol. In addition, ENBREL or other IL-1 receptor mimic may be administered in combination with methotrexate, sulfasalazine, gold salts, azathioprine, cyclosporine, antimalarials, oral steroids (e.g., prednisone) or colchicine. Non-steroidal anti-inflammatories may also be coadministered with the TNFR mimic, including but not limited to: salicylic acid (aspirin); ibuprofen; indomethacin; celecoxib; rofecoxib; ketorolac; nambumetone; piroxicam; naproxen; oxaprozin; sulindac; ketoprofen; diclofenac; and other COX-1 and COX-2 inhibitors, salicylic acid derivatives, propionic acid derivatives, acetic acid derivatives, fumaric acid derivatives, carboxylic acid derivatives, butyric acid derivatives, oxicams, pyrazoles and pyrazolones, including newly developed anti-inflammatories.

Moreover, the soluble type II IL-1 receptor may be used to treat psoriasis in combination with topical steroids, systemic steroids, antagonists of inflammatory cytokines, antibodies against T cell surface proteins, anthralin, coal tar, vitamin D3 and its analogs (including 1,25-dihydroxy vitamin D3 and calcipotriene), topical retinoids, oral retinoids (including but not limited to etretinate, acitretin and isotretinoin), topical salicylic acid, methotroxate, cyclosporine, hydroxyurea and sulfasalazine. In addition, it may be administered in combination with one or more of the following compounds; minocycline; misoprostol; oral collagen; penicillamine; 6-mercaptopurine; nitrogen mustard; gabapentin; bromocriptine; somatostatin; peptide T; anti-CD4 monoclonal antibody; fumaric acid; polyunsaturated ethyl ester lipids; zinc; and other drugs that may be used to treat psoriasis.

Psoriasis moreover may be treated by soluble type II IL-1 receptor administered in combination with one or more of the following topically applied compounds: oils, including fish oils, nut oils and vegetable oils; aloe vera; jojoba; Dead Sea salts; capsaicin; milk thistle; witch hazel; moisturizers; and Epsom salts.

In addition, psoriasis may be treated by soluble type II IL-1. receptor in combination with the following therapies: plasmapheresis; phototherapy with ultraviolet light B; psoralen combined with ultraviolet light A (PUVA); and sunbathing.

It is understood that the response by individual patients to the aforementioned medications or combination therapies may vary, and the most efficacious combination of drugs for each patient will be determined by his or her physician.

## Claims

1. An IL-1 inhibitor for use in treating a patient afflicted with a pulmonary disorder.

2. The IL-1 inhibitor of claim 1, wherein the pulmonary disorder is asthma or chronic obstructive pulmonary disease.

3. The IL-1 inhibitor of claim 1 or claim 2, which is an antibody directed against IL-1 or IL-1 beta or IL-1 receptor type I.

4. The IL-1 inhibitor of claim 1 or claim 2, which is IL-1Ra.

5. The IL-1 inhibitor of any preceding claim, which is for administration in combination with a TNF inhibitor.

6. The IL-1 inhibitor of any preceding claim, which is for administration in combination with a compound selected from the group consisting of antagonists of IFNγ, TGFβ, IL-6 and IL-8.

7. The use of an IL-1 inhibitor in the manufacture of a medicament for treating a patient afflicted with a pulmonary disorder.

8. The use of claim 7, modified by the features of any one of claims 2 to 6.
